# EUROPEAN PATENT APPLICATION

(11) **EP 3 878 508 A1**
(43) Date of publication of application: **15.09.2021**
(21) Application number: 20189475.5
(22) Date of filing: 04.08.2020
(51) Int. Cl.: A61N 5/06, H01S 3/095, H01S 3/223

(54) **CANCER TREATMENT SYSTEM**

(30) Priority: 13.03.2020 JP 2020044561
(71) Applicant: Takehisa, Kiwamu, Kawasaki-shi, Kanagawa 211-0004 (JP)
(72) Inventor: Takehisa, Kiwamu, Kawasaki-shi, Kanagawa 211-0004 (JP)
(74) Representative: Pfenning, Meinig & Partner mbB

(57) **Abstract**

The disclosure relates to a cancer treatment system including: a radiation source configured to produce 1.27-micrometer wavelength radiation, wherein the 1.27-micrometer wavelength radiation is generated from singlet oxygen. The radiation source may be an oxygen laser or an amplified spontaneous emission generator. The 1.27-micrometer wavelength radiation may be a laser or an amplified spontaneous emission.

## Description

### BACKGROUND

The present invention relates to a cancer treatment system.

There are many methods for treating cancer. Among them, a photodynamic therapy ("PDT") is well known. This therapy uses a photosensitizing drug that is injected intravenously. The photosensitizing drug is localized in diseased tissue. The photosensitizing drug is activated by light with a wavelength of from 0.63 to 0.66 micrometer. The molecules consisting of the drug (hereinafter referred to as drug molecules) are excited to become singlet state. But they are rapidly deactivated so as to be in their triplet state (This is called intersystem crossing).

Meanwhile, oxygen is dissolved in human tissue which could include cancer cells. Therefore, the dissolved oxygen near the cancer cells is transferred from the drug molecules in the triplet state to the excited singlet state O₂(¹Δ_{g}) by energy transfer. The oxygen in the excited singlet state is commonly called reactive oxygen and can destroy cells. Thus, cancer cells that collide with reactive oxygen species die. PDT is disclosed in, for example, Non-Patent literature 1 (Precision Engineering, The Japan Society for Precision Engineering, Vol. 81, No. 4, pp. 298-302).

On the other hand, a treatment apparatus based on a treatment method of generating a laser beam of a wavelength of 1.2 to 1.3 um and irradiating the laser beam directly to a cancer cell has been proposed. The laser light of this wavelength may excite the oxygen molecules around the cancer cells to be in a singlet state and may cause the cancer cells to die. This treatment has an advantage that it does not require a photosensitizing drug, which brings no adverse reaction. Published Japanese Translation of PCT International Publication for Patent Application, No. 2007-517559 discloses the treatment method and the treatment apparatus based on a laser beam of a wavelength of from 1.2 to 1.3 um.

In the above-mentioned treatment apparatus, a laser beam of a wavelength of from 1.2 to 1.3 um is generated by a Raman laser based on Raman conversion. However, in a method of generating a laser beam of a specific wavelength based on such wavelength conversion, in order to absorb the laser beam into oxygen molecules, it is necessary to precisely match the center wavelength of the laser beam to any one of a number of absorption lines in the 1.27 um band of oxygen molecules. Also, in order to efficiently absorb the laser radiation, it is necessary to narrow the wavelength width of the laser light to be equal to or less than the line width of the absorption line. Therefore, a line narrowing module and a wavelength stabilization module are necessary for the laser system, which makes the laser system complicated and expensive.

Alternatively, when the laser beam is generated with a wide wavelength width of about ± 0.5 nm covering the entire area around the wavelength 1.27 um without matching the laser wavelength to one of the absorption lines, many absorption lines are included. However, since the power component at a wavelength other than the absorption lines is overwhelmingly larger, there is a problem that the efficiency of singlet oxygen generation becomes extremely poor.

Meanwhile, Non-Patent Literature 7 ("Laser-induced generation of singlet oxygen and its role in the cerebrovascular physiology", Progress in Quantum Electronics Vol. 55, 112-128 (2017)" and Non-Patent Literature 8 ("Infrared laser pulse triggers increased singlet oxygen production in tumor cells", Scientific Reports, December 12, 2013) disclose that a quantum dot laser, which can be adjusted to oscillate at a 1.27-micrometer wavelength, is used to generate singlet oxygen. The quantum dot laser produces a laser with a linewidth of around 1 nm, which is much wider than the linewidth of any oxygen absorption line. Therefore, similar to the above described case, the efficiency of singlet oxygen generation becomes quite low.

### SUMMARY

An object of the present disclosure is to provide a cancer treatment apparatus using a light source capable of efficiently exciting oxygen molecules to become singlet oxygen.

In order to achieve the above object, the cancer treatment apparatus according to the embodiment includes a light source that generates light having a wavelength of 1.27 um, this light having a wavelength of 1.27 um being generated from singlet oxygen molecules. The cancer cells are irradiated with light having a wavelength of 1.27 um. The singlet oxygen molecules emit near-infrared light (hereinafter referred to as 1.27 um wavelength radiation or 1.27-micormeter wavelength radiation) with a wavelength of 1.27 um. More specifically, the singlet oxygen molecule has an emission spectrum including dozens of emission lines each having a wavelength near 1.268 um. This spectrum matches the absorption spectrum of the oxygen molecule in the ground state. In other words, the oxygen molecule in the singlet state emits a photon corresponding to the energy of its excited level, and the oxygen molecule in the ground state strongly absorbs the photon. Strong absorption means that the absorption cross section is relatively large. Therefore, by irradiating the periphery of the cancer cell with 1.27 um wavelength radiation, the dissolved oxygen existing in the vicinity of the cancer cell is efficiently excited into the singlet state.

Non-Patent literature 2 ("The Einstein coefficient for spontaneous emission of the O2(a1Δg) state", Geophysical Research Letters, Vol. 22, No. 11, pp. 1381 - 1384, 1995) and 3 ("Intensities of lines in the band a 1Δg (v '= 0) -X3 ∑g- (v" = 0) of 16O2 in absorption " Spectrochimica Acta, Vol. 48 A, No. 9, pp. 1227 -1230, 1992) disclose the absorption lines of oxygen molecule. In the Non-Patent literature 3, the absorption lines have a wavenumber of 788373 - 792080 m⁻¹, which corresponds to the wavelength of 1.2625 to 1.2684 micrometer. The reason why there are a large number of absorption lines in oxygen molecules is that, in both the ground and singlet states of oxygen molecules, their electronic states are separated into a large number of rotational levels due to the diatomic nature of the oxygen molecule.

In particular, by using a laser beam oscillated from an oxygen molecule as the 1.27 um wavelength radiation, unlike the spontaneous emission light, the light condensing property of light condensed by the condenser lens can be enhanced. Therefore, it becomes easy for light to enter the optical fiber, and flexible transmission using the optical fiber becomes possible. This enables an optical fiber to be used in the optical system, and thus flexible transmission can be realized. For example, Non-Patent literature 4 ("Possible high energy laser at 1.27 um", Applied Optics, Vol. 17, No. 20, pp. 3276 - 3283, 1978), Non-Patent literature 5 ("Chemically Pumped O2(a-X) Laser", Applied Physics B, Vol. 56, pp. 71 - 78, 1993) and Non-Patent literature 6 ("New concepts of realizing a chemical oxygen laser", Proceedings of SPIE, Vol. 9251, pp. 9251X-1 - 9251X-15, 2014) disclose the oxygen molecular laser.

Singlet oxygen, as the source of 1.27-micrometer wavelength radiation, can be generated by the discharge of oxygen, such as silent discharge or microwave discharge, but is preferably generated by the chemical reaction of BHP (Basic Hydrogen Peroxide) solution and chlorine gas. The BHP solution is a solution of an alkaline solution such as potassium hydroxide mixed with hydrogen peroxide solution.

The reason why the above chemical reaction is used is that a large number of chemicals, prepared beforehand, can be instantly reacted and thus the power of the 1.27-micrometer wavelength radiation can be enhanced easily. When a high-power 1.27 um wavelength radiation is obtained, treatment can be carried out in a short time. Alternatively, by branching the 1.27 um wavelength radiation into many beams, not only can the treatment time be shortened, but it is also possible to treat many patients simultaneously.

As described above, it is difficult to efficiently generate laser light having a wavelength that matches the absorption line of oxygen molecules at a wavelength of 1.27 um from a laser device other than the oxygen molecule laser. Therefore, in the present embodiment, it is preferable to use an oxygen molecule laser as a light source for the 1.27 um wavelength radiation. However, instead of an oxygen molecule laser, amplified spontaneous emission (ASE) can be used as the source of the 1.27-micrometer wavelength radiation. ASE stands for Amplified Spontaneous Emission. For example, by filling the elongated region with singlet oxygen molecules generated based on the chemical reaction described above, the amplified spontaneous emission emitted from the longitudinal direction of the region can be used.

The amplified spontaneous emission mentioned in the present disclosure is made to emit radiation) strongly only in a specific direction, which is different from the spontaneous emission light that emits radiation isotropically in every direction. According to this method, there is a case where spontaneous emission generated inside of the laser cavity is returned by making it enter one of the two reflecting mirrors, and then it collides with singlet oxygen molecules to emit stimulated emission.

The present invention provides a cancer treatment system using a light source which can efficiently generate singlet oxygen.

The above and other objects, features and advantages of the present invention will become more fully understood from the detailed description given herein below and the accompanying drawings which are given by way of illustration only, and thus are not to be considered as limiting the present invention.

### BRIEF DESCRIPTION OF THE DRAWINGS

Fig. 1 is a schematic diagram showing a cancer treatment apparatus according to Embodiment 1;
Fig. 2 is a graph showing skin permeability;
Fig. 3 is a schematic diagram showing the configuration of an oxygen molecule laser;
Fig. 4 is a cross-sectional view schematically showing the configuration of an excited oxygen generator used in an oxygen molecule laser;
Fig. 5 is a schematic diagram showing a cancer treatment apparatus according to Embodiment 2;
Fig. 6 is a schematic diagram showing the configuration of a spontaneous emission amplified light generator;
Fig. 7 is a schematic diagram showing a configuration of a spontaneous emission amplified light generator according to a third embodiment;
Fig. 8 is a cross-sectional view schematically showing the configuration of a spontaneous emission amplification light generator according to a third embodiment; and
Fig. 9 is a general view schematically showing a configuration of a spontaneous emission amplification light generator according to a fourth embodiment.

### DESCRIPTION OF EMBODIMENTS

Exemplary embodiments of the present invention are explained with reference to the attached drawings. The exemplary embodiments explained below are examples of the present invention, and the present invention is not limited to these exemplary embodiments. Note that components denoted by the same reference numerals in the specification and drawings indicate the same components.

### First Embodiment

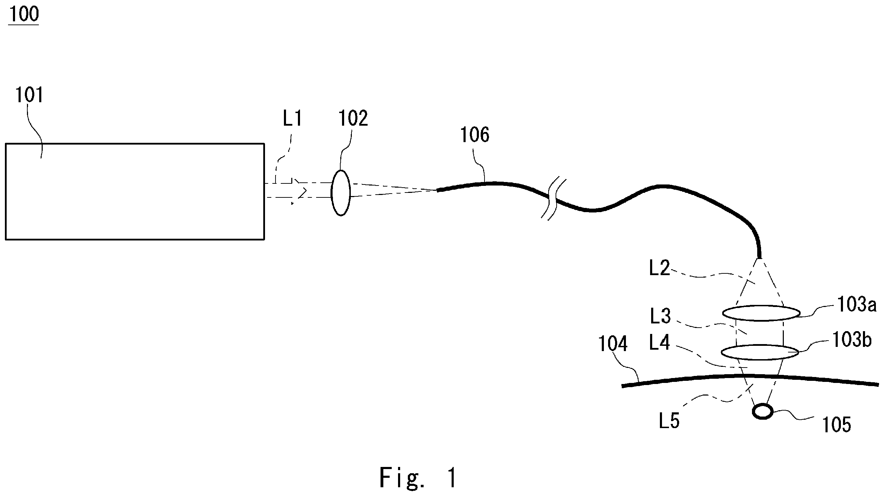

Hereinafter, the first embodiment according to the present invention is described based on Fig. 1. Fig. 1 is a block diagram showing the cancer treatment system 100 according to the present invention. The cancer treatment system 100 has an oxygen laser oscillator 101 from which a laser beam L1 is extracted. The oxygen laser oscillator 101 is a light source for generating a laser beam L1 having a wavelength of 1.27 um. The oxygen laser oscillator 101 is placed outside the patient. The laser beam L1 emitted from the oxygen laser oscillator 101 passes a focusing lens 102, which focuses the laser beam L1 at the input end of an optical fiber 106. Accordingly, the laser beam L1 is incident on the optical fiber 106 made of quartz. After the laser beam L1 is propagated in the optical fiber 106, the laser beam is extracted from the output end of the optical fiber 106 which is located near the patient to be treated.

The laser beam L2 emitted from the optical fiber 106 travels while spreading and enters a focusing lens 103a. The beam diameter of the laser beam L2 is enlarged to about several centimeters and it becomes the laser beam L3. After the laser beam L3 goes through the focusing lens 103b, it becomes a focusing beam L4.

The focusing lens 103b is placed close to the skin 104 of the patient. The focusing beam L4 focused by the focusing lens 103b is made incident on the skin 104 immediately after the focusing lens 103b placed close to the skin of the patient. Since the beam diameter of the focusing beam L4 on the skin is a few centimeters, the beam intensity is low. After the laser beam (laser beam L5) goes through the skin, it is most strongly focused at a cancer cell 105. The laser beam L5 is incident on the cancer cell 105 through the skin 104. Consequently, many dissolved oxygen molecules are excited and become active oxygen (singlet oxygen). The converging beam L5 is incident on the cancer cell 105. The active oxygen then kills the cancer cell 105.

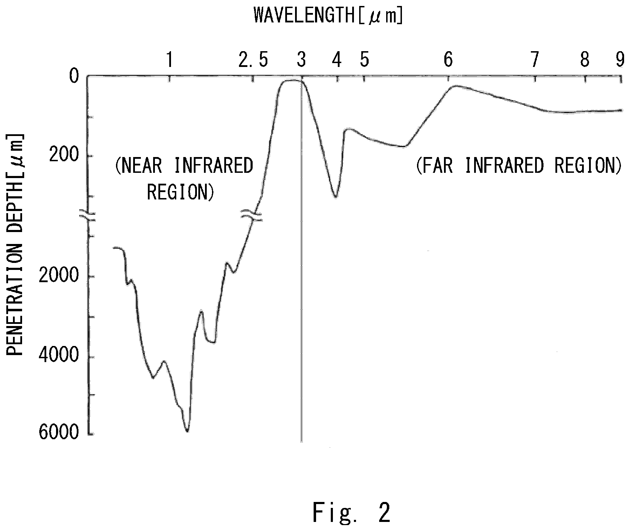

Fig. 2 shows the transmissivity of human skin. Fig. 2 is disclosed in the Non-Patent literature 9 ("Spectral radiative properties of a living human body", International Journal of Thermophysics, Vol. 7. pp.1101 - 1113, 1986). As is shown in Fig. 2, the radiation at a 1.27-micrometer wavelength has a relatively high transmissivity. Consequently, it can penetrate deeply into the human body. As mentioned above, the intensity of the beam before it reaches the cancer cell 105 can be low. This enables the amount of the active oxygen from skin to the cancer cell to be suppressed.

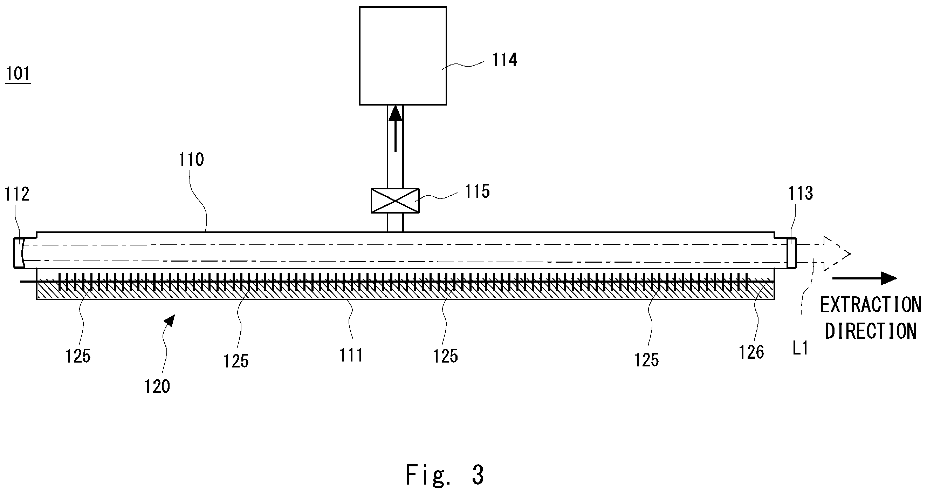

The oxygen laser oscillator 101 is explained with reference to Fig. 3. The oxygen laser oscillator 101 has a laser cavity 110 and a singlet oxygen generator (SOG) 120. The laser cavity 110 and a SOG housing 111 are placed close to each other. The SOG housing 111 is located directly below the laser cavity 110. The axis of the laser cavity 110 (horizontal direction in Fig 3) is parallel to the longitudinal direction of the SOG housing 111.

A plurality of rotating disks 125 are provided in the housing 111. The rotating disks 125 can rotate around a rotation axis 126. The direction of the rotation axis 126 is parallel to the longitudinal direction of the SOG housing 111. The plurality of the rotating disks are placed parallel to each other. The plurality of the rotating disks are arranged along the longitudinal direction. The details of the SOG including the SOG housing 111 are explained later.

A total reflection mirror 112 is attached at one end of the laser cavity 110. An output mirror 113 is attached at the other end of the laser cavity 110. The total reflection mirror 112 has 99.9 % reflectivity at the wavelength of 1.27 micrometer. The output mirror 113 has 99.0 % reflectivity at the wavelength of 1.27 micrometer. The laser beam L1 is extracted from the output mirror 113 in an extraction direction (rightward direction). A vacuum pump 114 is connected to the laser cavity 110 through a valve 115.

Before the laser operation, the laser cavity 110 is evacuated by the vacuum pump 114. To start lasing, the valve 115 is closed. Singlet oxygen is generated in the SOG housing 111, and then the laser cavity is filled with singlet oxygen. This increases the pressure of singlet oxygen in the laser cavity. When the pressure reaches the threshold value, a pulsed laser beam L1 is extracted from the output mirror 113. After the lasing, the valve 115 is opened. The vacuum pump then evacuates the inside of the laser cavity 110 to prepare for the next laser operation.

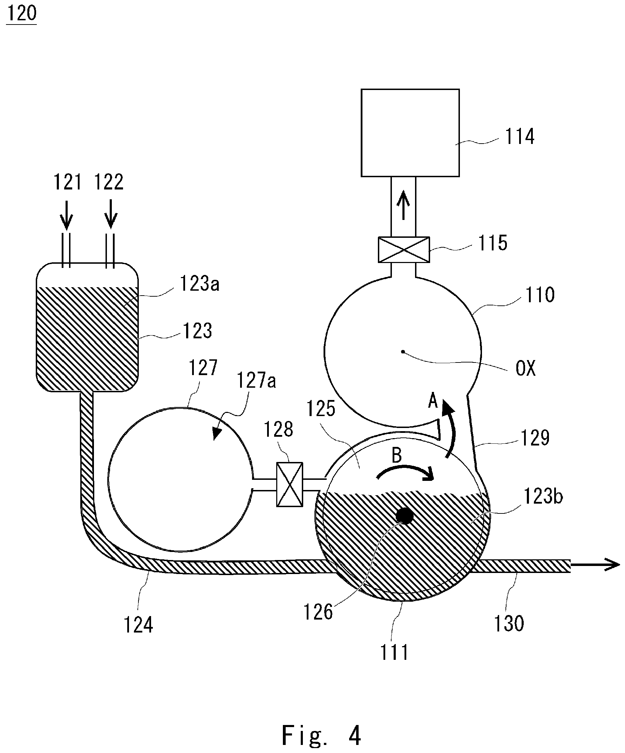

The inside of the SOG 120 is explained with reference to Fig. 4. Fig. 4 is a cross-sectional drawing of the SOG 120. Fig. 4 shows a cross sectional plane perpendicular to the optical axis of the laser cavity 110. That is, Fig. 4 shows a cross sectional plane perpendicular to the longitudinal direction of the SOG housing 111.

The SOG 120 has a BHP tank 123. Hydrogen peroxide 121 and potassium hydroxide 122 are supplied to the BHP tank 123 and then are mixed to be a BHP solution 123a in the BHP tank 123. The BHP solution 123a is a necessary chemical solution for enabling a chemical reaction to generate singlet oxygen. In this way, the BHP solution 123a is stored in the BHP tank 123 before the laser operation.

The BHP solution 123a is supplied from the BHP tank 123 to the SOG housing 111 through the supply pipe 124. The SOG housing 111 stores the BHP solution 123a. The SOG housing 111 has a cylindrical shape with its longitudinal direction being perpendicular to this drawing. The plurality of rotating disks 125 are located in the SOG housing 111 (see Fig. 3). The rotation axis 126 is located at the center of the rotating disks 125.

Before the chemical reaction, the rotating disks 125 in the SOG housing 111 rotate in a direction as indicated by an arrow B. In this way, the BHP solution 123b is adhered to the entire surface of the rotating disks 125.

A chlorine gas cylinder 127 is connected to the SOG housing 111 through a valve 128. The chlorine gas cylinder 127 is filled with a chlorine gas 127a. The chlorine gas 127a is a gas necessary for enabling the chemical reaction. In this way, the chlorine gas 127a is stored in the chlorine gas cylinder 127 before the laser operation.

In order to generate singlet oxygen, the valve 128 is opened. Then the chlorine gas 127a is supplied into the SOG housing 111. Consequently, the BHP solution 123b on the surface of the rotating disks 125 starts to react with the chlorine gas 127a. This chemical reaction generates singlet oxygen.

The SOG housing 111 is connected to the laser cavity 110 through a duct 129. The singlet oxygen generated in the SOG housing 111 is supplied to the laser cavity 110 as indicated by an arrow A. When the pressure of the singlet oxygen filling the laser cavity 110 exceeds a threshold value, the lasing starts. Then, the laser beam L1 is extracted from the laser cavity 110 as shown in Fig. 3. The BHP solution 123b used in the chemical reaction is then drained from the drainpipe 130.

By employing the oxygen laser oscillator 101 as the radiation source, it is possible to efficiently generate the 1.27-micrometer wavelength radiation. The oxygen laser oscillator 101 produces the laser beam L1 based on the stimulated emission from singlet oxygen. By using the laser emitted from the singlet oxygen, it is possible to efficiently treat the cancer. Since the emission lines of singlet oxygen exactly match the absorption lines of ground state oxygen, it is possible to efficiently treat the cancer.

### Second embodiment

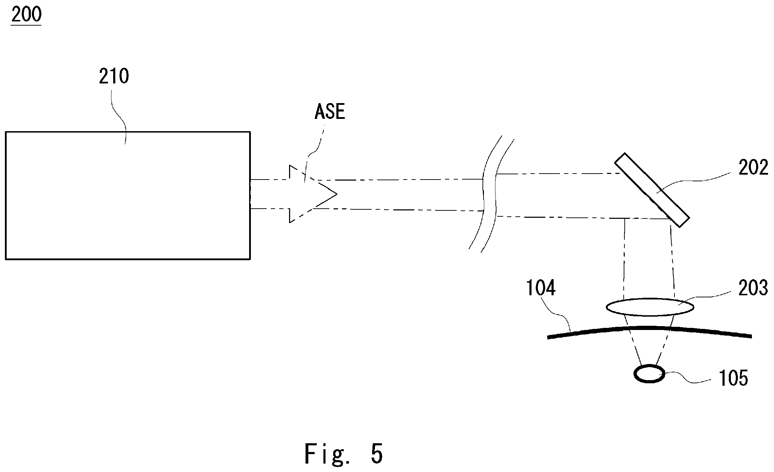

The second embodiment according to the present invention will be described with reference to Fig. 5. Fig. 5 is a diagram of a cancer treatment system 200 in the second embodiment. The cancer treatment system 200 employs an amplified spontaneous emission (ASE) generator 210, instead of the laser cavity 110 of the first embodiment, as the radiation source. Since the amplified spontaneous emission has a poorer focusing characteristic than that of a laser, power delivery using an optical fiber is difficult. Therefore, the optics of cancer treatment system 200 is different from that of the cancer treatment system 100 in the first embodiment. Specifically, an optical fiber is not employed in the system.

The ASE generator 210 is the source for generating 1.27-micrometer wavelength radiation. The amplified spontaneous emission ASE extracted from the ASE generator 210 is expandingly propagated toward a mirror 202. The amplified spontaneous emission ASE enters the mirror 202. The mirror 202 is placed above the diseased part of a patient. The amplified spontaneous emission ASE is reflected downwardly by the mirror 202. The amplified spontaneous emission ASE reflected by the mirror 202 propagates toward the affected part. The amplified spontaneous emission ASE reflected by the mirror 202 is focused by the lens 203. The amplified spontaneous emission ASE becomes a converging beam and then the converging amplified spontaneous emission ASE travels to the skin 104 of the patient. The converging amplified spontaneous emission ASE passes through the skin 104, and is focused around the cancer cell 105. Consequently, many dissolved oxygen molecules are excited and become active oxygen (singlet oxygen). Then the active oxygen kills the cancer cell 105.

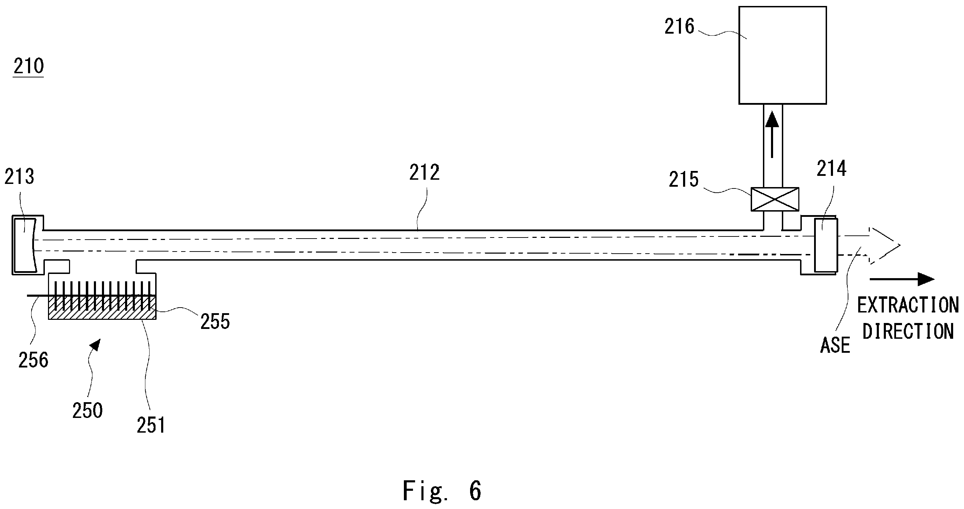

The configuration of the ASE generator 210 is explained with reference to Fig. 6. The ASE generator 210 has a SOG 250 and a metal pipe 212. The SOG 250 has a SOG housing 251, rotating disks 255 and the rotation axis 256. The SOG housing 251, the rotating disks 255 and the rotation axis 256 correspond to the SOG housing 111, the rotating disks 125 and the rotation axis 126 shown in Fig. 3 and Fig. 4, respectively. Since the configuration of the SOG 250 is the same as that of the SOG 120 as shown in the first embodiment, the explanation thereof is omitted. Namely, the SOG 250 has the same structural configuration as that of the SOG 120. Therefore, the supply of the BHP solution and chlorine gas is not shown in Fig. 6.

A long narrow metal pipe 212 is located just above the SOG 250. The metal pipe 212 is a cylindrical container. The horizontal direction of Fig. 6 corresponds to the longitudinal direction (axis direction) of the metal pipe 212. The singlet oxygen generated in the SOG 250 is supplied into the metal pipe 212 until it fills it.

The metal pipe 212 is made of stainless (SUS316). The inner surface of the metal pipe 212 is electropolished. The metal pipe 212 with an electropolished surface is commonly called an EP tube. A high reflection mirror 213 is provided at one end of the metal pipe 212 and a window 214 is provided on the other end of the metal pipe 212.

The high reflection mirror 213 has about 99.9 % reflectivity at a wavelength of 1.27 micrometer. The window 214 is made of transparent silica glass. The window 214 transmits the 1.27-micrometer radiation.

The singlet oxygen in the metal pipe 212 produces spontaneous emission in every direction. However, some parts of the spontaneous emission are reflected by the high reflection mirror 213, and thus go toward the window 214. Therefore, the power of the radiation propagated toward the window 214 (rightward direction in Fig. 6) is increased. Consequently, the spontaneous emission extracted from the window 214 becomes the amplified spontaneous emission ASE.

A vacuum pump 216 is connected to the metal pipe 212 through a valve 215. After the extraction of the amplified spontaneous emission ASE, the valve 215 opens. Then, the inside of the metal pipe 212 is evacuated for the next operation. The valve 215 can stay open during the operation. In this case, the amplified spontaneous emission ASE is extracted continuously, though its power is low.

The reason for using an electropolished pipe as the metal pipe 212 is explained below. The singlet oxygen inside the metal pipe 212 produces spontaneous emission in every direction. Namely, the 1.27-micrometer wavelength radiation is emitted in every direction. Therefore, some parts of the spontaneous emission propagate toward the inner side surface of the metal pipe 212. The spontaneous emission is reflected at the inner side surface of the metal pipe 212. Consequently, the reflected radiation can be absorbed in the ground-state oxygen. Then the oxygen can be excited to become singlet oxygen. This means that the spontaneous emission propagating toward the inner side surface of the metal pipe 312 can be used to generate singlet oxygen. Therefore, the spontaneous emission propagating toward the window 214 can be enhanced. The spontaneous emission becomes the amplified spontaneous emission ASE, and the amplified spontaneous emission ASE can be extracted from the window 214.

### Third embodiment

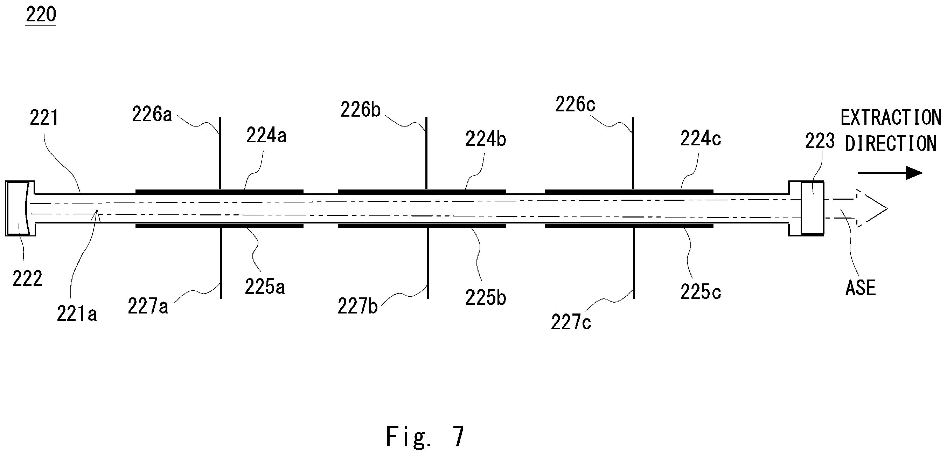

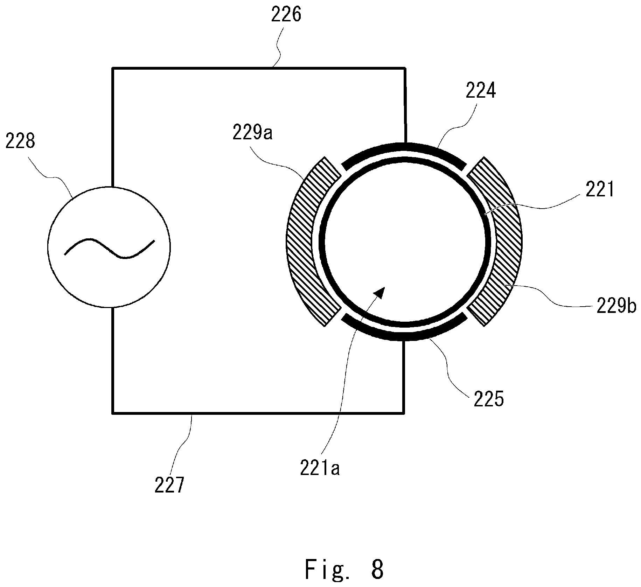

In a third embodiment, an ASE generator has a configuration different from that of the ASE generator 210 according to the second embodiment. In the third embodiment, the system configuration other than the ASE generator is the same as that of the second embodiment, and the explanation thereof is thus omitted. The ASE generator 220 according to the third embodiment will be described with reference to Figs. 7 and 8. Fig. 7 shows the ASE generator 220 in a cross-sectional view along the axis direction of the ASE generator 220. Fig. 8 shows the ASE generator 220 in a cross-sectional view along a plane perpendicular to the axis direction of the ASE generator 220.

As shown in Fig. 7, the ASE generator 220 includes a silica-glass pipe 221, electrodes 224a to 224c, electrodes 225a to 225c, electrical wires 226a to 226c, and electrical wires 227a to 227c. In this embodiment, an SOG is not employed in the system. The ASE generator 220 is a radiation source to produce 1.27-micrometer wavelength radiation.

As shown in Fig. 7, three sets of the electrodes 224a to 224c and the electrical wires 226a to 226c are arranged in series. The electrodes 224a to 224c and electrical wires 226a to 226c are shown together as the electrode 224 and the electrical wire 226, respectively in Fig. 8. For example, the electrode 224a is connected with the electrical wire 226a. The electrode 224b is connected with the electrical wire 226b. The electrode 224c is connected with the electrical wire 226c. Similarly, three sets of the electrodes 225a to 225c and the electrical wires 227a to 227c are arranged in series. The electrodes 225a to 225c and the electrical wires 227a to 227c are shown together as the electrode 225 and the electrical wire 227, respectively in Fig. 8.

The silica-glass pipe 221 is a cylindrical container. The longitudinal direction (axis direction) of the silica-glass pipe 221 is parallel to the radiation extraction direction. The inside of silica-glass pipe 221 is filled with oxygen 221a. Singlet oxygen is generated from the inside oxygen 221a by silent discharge.

The electrode 224 and electrode 225 are contacted with the outside surface of the silica-glass pipe 221. The three electrodes 224a to 224c are positioned along the longitudinal direction of the silica-glass pipe 221. The electrodes 224a to 224c are spaced from each other and are positioned in a row. Similarly, the three electrodes 225a to 225c are positioned along the longitudinal direction of the silica-glass pipe 221. The electrodes 225a to 225c are spaced from each other and are positioned in a row.

The electrode 224 and the electrode 225 are opposed to each other with the silica-glass pipe 221 interposed therebetween. Namely, the electrodes 224 are positioned just above the silica-glass pipe 221, and the electrodes 225 are positioned just below the silica-glass pipe 221. The position and the length of the electrode 224a are the same as those of the electrode 225a.

The electrodes 224 are connected to a high-frequency power supply 228 through the electrical wires 226. The electrodes 225 are connected to the high-frequency power supply 228 through the electrical wires 227. The high-frequency power supply 228 produces pulsed high voltage at about 10 kHz. The high-frequency power supply 228 provides the high-frequency voltage to the electrodes 224 and the electrodes 225. The high-frequency voltage applied to the electrodes 224 and the electrodes 225 generates discharge to oxygen molecules. Then, the oxygen is excited by the discharge, and the singlet oxygen is generated.

An insulator 229a and an insulator 229b are contacted to the outer surface of the silica-glass pipe 221. The insulator 229a and the insulator 229b can suppress the surface discharge between the electrode 224 and the electrode 225. The insulator 229a and the insulator 229b are positioned between the electrode 224 and the electrode 225 in the circumferential direction of the silica-glass pipe 221. The insulator 229a is positioned at the left of the silica-glass pipe 221, and the insulator 229b is positioned at the right of the silica-glass pipe 221 as shown in Fig. 8.
The electrode 224, the insulator 229a, the electrode 225 and the insulator 229b are positioned in this order in the circumferential direction of the silica glass pipe 221.

The insulator 229a and the insulator 229b are positioned on the outer surface of the silica-glass pipe 221. This enables the surface discharge generated along the side surface of the silica-glass pipe 221 to be suppressed. Unlike the electrode 225 and the electrode 226, the insulator 229a and the insulator 229b are not divided into three parts. The insulator 229a and the insulator 229b are attached on the entire surface of the silica-glass pipe 221 in the longitudinal direction. Namely, the insulator 229a, as a single long part, is attached on the side surface of silica-glass pipe 221.

A high reflection mirror 222 is attached at one end of the silica-glass pipe 221. A window 223 is attached at the other end of the silica-glass pipe 221. The window 223 is made of silica glass which can transmit 1.27-micrometer wavelength radiation. This enables the extraction of 1.27-micrometer wavelength radiation from the window 223. Therefore, the amplified spontaneous emission ASE is generated by the discharge of the oxygen and is extracted from the window 223.

A dielectric multilayer coating is provided on the inner surface of the silica-glass pipe 221. The dielectric multilayer coating has a high reflectivity at the wavelength of 1.27 micrometer. This enables 1.27-micromenter wavelength spontaneous emission to suppress transmission of 1.27-micrometer wavelength radiation through the side surface of the silica-glass pipe 221. In brief, the multilayer coating has the same function as that of the metal pipe 212 shown in Fig. 5. Concretely, among the spontaneous emissions emitting in every direction, some of them having the propagation direction toward the window 223 are enhanced. Consequently, the amplified spontaneous emission ASE is extracted.

### Fourth embodiment.

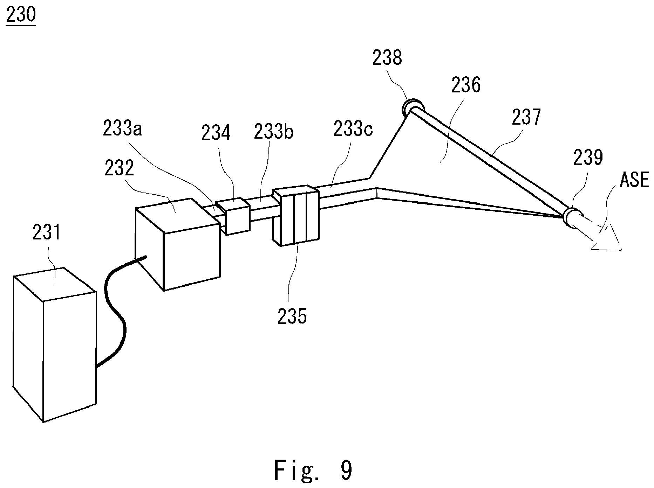

In the fourth embodiment, the configuration of the ASE generator is different from those of the second and third embodiments. The ASE generator according to the embodiment will be described with reference to Fig. 9. Fig. 9 is a perspective drawing of the ASE generator 230. The ASE generator 230 generates singlet oxygen by microwave.

The ASE generator 230 has a power supply 231, a microwave oscillator 232, a waveguide 233a, an isolator 234, a waveguide 233b, a tuner 235, a tapered waveguide 236, and a silica-glass pipe 237. The ASE generator 230 is a radiation source for generating 1.27-micometer wavelength radiation.

The power supply 231 supplies operating voltage to the microwave oscillator 232. The microwave oscillator generates the microwave. The microwave generated in the microwave oscillator 232 is transmitted to the isolator 234 through the waveguide 233a. The microwave from the isolator 234 is transmitted to the tuner 235 through the waveguide 233b. The microwave from the tuner 235 is transmitted to the tapered-waveguide 236 through the waveguide 233c.

The exit end of the tapered-waveguide 236 is connected to a silica-glass pipe 237. The width of the tapered-waveguide 236 gradually increases toward the exit end. The silica-glass pipe 237 is a cylinder like the silica-glass pipe 221 shown in the third embodiment. Since the tapered-waveguide 236 has an expanding width toward the exit end in the longitudinal direction (axis) of the silica-glass pipe 237, the microwave from the tapered-waveguide 236 is provided to the whole area of the silica-glass pipe 237 in the longitudinal direction.

The silica-glass pipe 237 is a container filled with oxygen. When a microwave is provided from the tapered-waveguide 236, oxygen is discharged. Consequently, like in the case of the third embodiment, singlet oxygen is generated.

The silica-glass pipe 237 has a configuration similar to that of the silica-glass pipe 221 shown in the third embodiment. For example, a high reflection mirror 238 is attached at one end of the silica-glass pipe 237. A window 239 is attached at the other end of the silica-glass pipe 237. The window 239 is made of silica glass. The 1.27-micrometer radiation can be transmitted through the window 239.

The high reflection mirror 238 has a high reflectivity at the wavelength of 1.27 micrometer. Therefore, spontaneous emission from singlet oxygen generated in the silica-glass pipe 237 is enhanced along the longitudinal direction of the silica-glass pipe 237.
Consequently, an amplified spontaneous emission ASE with the wavelength of 1.27 micrometer is extracted from the window 239.

An advantage of this embodiment is the utilization of a microwave with a high-power output. Since a microwave is used in a radar, it can be generated at high power easily. Therefore, high power amplified spontaneous emission ASE can be obtained.

In the cancer treatment systems according to the embodiments 1 to 4, high-power 1.27-micrometer wavelength radiation is generated. Since the 1.27-micrometer wavelength light has a high transmissivity on human skin, it effectively treats not only skin cancer and stomach cancer that are present near the skin, but also other types of cancer which are present deep inside the body.

In the above embodiments 2 to 4, the amplified spontaneous emission ASE is used as the radiation. This enables the 1.27-micrometer wavelength radiation to be efficiently produced. Using the 1.27-micrometer wavelength radiation from singlet oxygen enables the cancer cells to be killed effectively. Since the spectra of the emission lines of singlet oxygen match the spectra of the absorption lines of ground-state oxygen, the cancer cells can be killed efficiently.

In the case of using ASE as the treatment radiation, the window 214, 223 or 239 can be placed close to the diseased part of the patient. This enables use of the extracted radiation, which is expanding at a large angle from the window.

In the above embodiments, the present cancer treatment system and a hyperbaric oxygen therapy are preferably performed simultaneously. More particularly, it is possible to obtain a high treatment effectiveness by using the cancer treatment system while performing the hyperbaric oxygen therapy when the diseased part is large. The hyperbaric oxygen therapy is a kind of therapy in which oxygen-based antimicrobial activity is enhanced by increasing the concentration of dissolved oxygen in a patient's blood, which is realized by placing the patient under about 2 atm air pressure. When the diseased part is irradiated by the 1.27-micrometer wavelength radiation from the cancer treatment system under high pressure air, for example 2atm, more singlet oxygen is generated from the increased number of oxygen molecules in the patient's blood. This greatly improves cancer cell killing effectiveness of killing cancer cell.

Some or all of the above embodiments can be combined as desirable by one of ordinary skill in the art. While the invention has been particularly shown and described with reference to exemplary embodiments thereof, the invention includes various modifications which do not negatively affect the purpose and advantages of the invention and is not limited to these exemplary embodiments.

From the invention thus described, it will be obvious that the embodiments of the invention may be varied in many ways. Such variations are not to be regarded as a departure from the spirit and scope of the invention, and all such modifications as would be obvious to one skilled in the art are intended for inclusion within the scope of the following claims.

## Claims

1. A cancer treatment system comprising:
a radiation source configured to produce 1.27-micrometer wavelength radiation,
wherein the 1.27-micrometer wavelength radiation is generated from singlet oxygen.

2. The cancer treatment system according to claim 1,
wherein the radiation source is an oxygen laser, and
the 1.27-micrometer wavelength radiation is extracted from the oxygen laser as a laser.

3. The cancer treatment system according to claim 2,
wherein the oxygen laser includes a singlet oxygen generator which can generate singlet oxygen,
the singlet oxygen is generated from a chemical reaction between a solution of alkaline solution mixed with hydrogen peroxide and chlorine gas, and
the laser is given by the stimulated emission from the singlet oxygen.

4. The cancer treatment system according to claim 1, wherein the 1.27-micrometer wavelength radiation is amplified spontaneous emission from singlet oxygen.

5. The cancer treatment system according to claim 4, further comprising:
a singlet oxygen generator configured to generate the singlet oxygen; and
a container configured filled with the singlet oxygen supplied from the singlet oxygen generator, and
a mirror attached at one end of the container in a longitudinal direction of the container and configured to reflect the 1.27-micrometer wavelength radiation,
wherein the singlet oxygen is generated from the chemical reaction between a solution of alkaline solution mixed with hydrogen peroxide and chlorine gas, and
an inner surface of the container reflects the 1.27-micrometer wavelength

6. The cancer treatment system according to any one of claims 1 to 5, wherein the radiation source is placed outside a patient.

7. The cancer treatment system according to any one of claims 1 to 6, further comprising:
a focusing lens configure to converge the 1.27-micrometer wavelength radiation,
wherein the converging 1.27-micrometer wavelength radiation is incident on cancer cells.

8. The cancer treatment system according to any one of claims 1 to 7, wherein the 1.27-micrometer wavelength radiation is incident on cancer cells through a skin of a patient.
